# EUROPEAN PATENT SPECIFICATION

(11) **EP 2 019 703 B1**
(45) Date of publication and mention of the grant of the patent: **12.01.2011**
(21) Application number: 07777178.0
(22) Date of filing: 18.05.2007
(51) Int. Cl.: A61M 25/06, A61M 5/158

(54) **IV catheter assembly with an ergonomic needle grip**
IV-Katheteranordnung mit ergonomischem Nadelgriff
Ensemble de cathéter intraveineux à élément de préhension d'aiguille ergonomique

(30) Priority: 22.05.2006 US 439481
(43) Date of publication of application: 04.02.2009
(73) Proprietor: Becton, Dickinson & Company, Franklin Lakes, NJ 07417-1880 (US)
(72) Inventor: HANNER, Gert, S-263 93 Mjohult (SE); HAGER, Jorgen, Bruno, S-25250 Helsingborg (SE); SODERHOLM, Karl Johan, Marten, S-256 58 Helsingborg (SE); GLOWACKI, Kristoffer, S-24532 Staffanstorp (SE)
(74) Representative: von Kreisler Selting Werner
(86) International application number: PCT/US2007/012037
(87) International publication number: WO 2007/139741

(56) References cited:
- EP-A- 0 034 879
- EP-A1- 0 606 695
- EP-A2- 0 281 421
- WO-A-02/096493
- WO-A-03/075995
- US-A- 3 595 230

## Description

### BACKGROUND OF THE INVENTION

The present invention relates generally to an ergonomic needle grip for a catheter assembly.

In order to properly place medical devices such as intravenous ("IV") catheters into a patient, the catheter is typically mounted over an introducer needle having a sharp distal tip. The distal tip of the needle preferably extends past the distal tip of the catheter. The catheter and introducer needle are inserted at a shallow angle through the patient's skin into a vein. Typically, the clinician confirms that there is a flashback of blood in a flashback chamber incorporated into the needle hub assembly in order to verify proper placement of the catheter in the vein. Once the catheter is properly inserted, the clinician then withdraws the needle, leaving the catheter in place for use in accordance with standard medical technique.

It is common for clinicians to use varying techniques for inserting a catheter into a patient. These varying techniques are due in part to comfort of the clinician, and in part to the fact that different types of catheters are available on the market. Two common types of catheters are straight and ported catheters. A ported catheter typically includes a radially extending side port integral with the catheter adapter. (See for example the catheter disclosed in U.S. Patent No. 5,098,405). Ported catheters are commonly used in Europe. With a ported catheter, the clinician typically grasps the assembly by placing the thumb on a grip plate, one finger on the side port of the catheter adapter, and another finger on a front edge of a catheter hub wing. Alternatively, the clinician may grasp the assembly by placing the thumb on the grip plate and the forefinger or middle finger on the side port of the catheter adapter. A straight catheter, on the other hand, does not include a side port for connection to a fluid handling device. Thus, the fluid handling device is instead connected to the proximal end of the catheter adapter. (See for example the catheters disclosed in U.S. Patent Nos. 4,193,400 and 5,685,855). Such straight catheters are typically used in the United States. With a straight catheter, the clinician typically grasps the assembly by placing the thumb and forefinger or middle finger of one hand on either side of the needle hub.

Once a clinician learns a particular technique to insert a catheter into a patient, that clinician will likely continue to use that technique to insert catheters into future patients. Problems arise when a clinician trained to insert a ported catheter must adjust to inserting a straight catheter, and vice versa.

WO 02/096493 discloses a catheter assembly of the type defined in the first part of claim 1. This catheter assembly comprises a catheter adapter, a needle hub and a tip shield. The catheter adapter has an injection port and the needle hub is provided with a grip plate. The grip plate extends in a right angle from the needle hub. The catheter assembly can be grasped between a thumb and a forefinger of a user's hand.

### SUMMARY OF THE INVENTION

It is an object of the invention to provide a catheter assembly with an ergonomic needle grip that enables the clinician to securely grasp the catheter, and that further allows for varying insertion and removal techniques.

The catheter assembly of the invention is defined by claim 1.

The present invention is a catheter assembly with an ergonomic needle grip attached to a needle hub of the catheter assembly. The needle grip comprises a grip plate attached to and extending radially from an outer surface of the needle hub. The grip plate forms an obtuse angle with an axis extending longitudinally along a centerline of a needle, wherein the angle is measured from a point on the axis near a proximal end of the needle hub. A flashback chamber of the catheter assembly located near the proximal end of the needle hub is visible when a digit is placed on the grip plate.

### BRIEF DESCRIPTION OF THE DRAWINGS

FIG. 1 is a perspective view of a catheter assembly of the present invention prior to insertion.
FIG. 2 is a perspective view of the catheter assembly of the present invention after insertion.
FIG. 3 is a perspective view of a needle grip of the present invention attached to a needle hub.
FIG. 4 is a side view of the catheter assembly of the present invention showing a first method of grasping the needle grip.
FIG. 5 is a perspective view of the catheter assembly of the present invention showing a second method of grasping the needle grip.
FIG. 6*a* is a side view of the needle grip with a grip plate at a first angle.

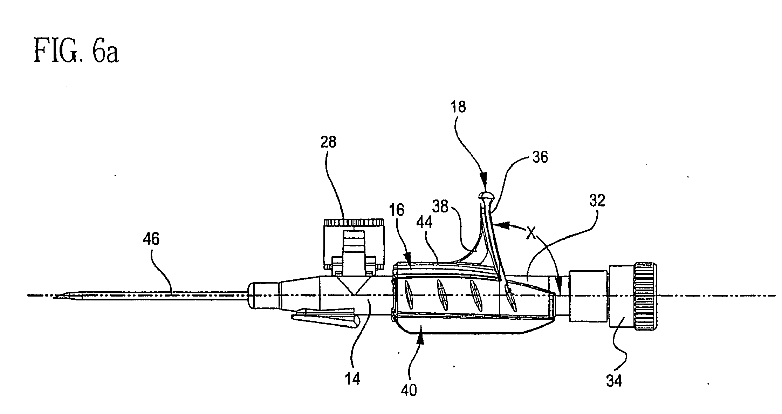

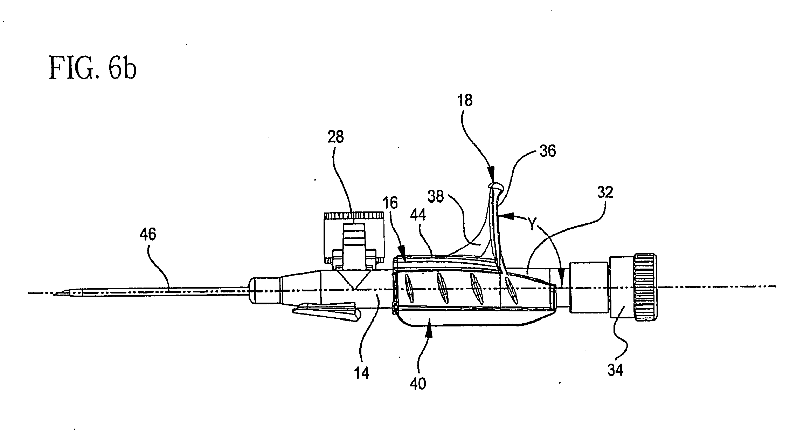

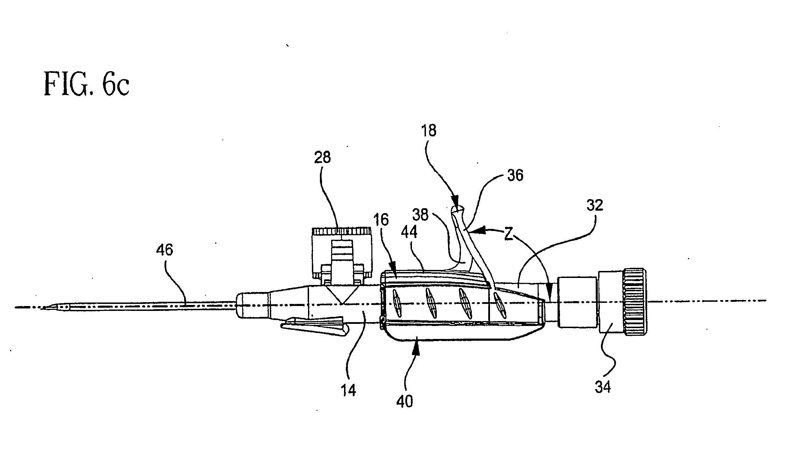

FIG. 6*b* is a side view of the needle grip with the grip plate at a second angle.
FIG. 6*c* is a side view of the needle grip with the grip plate at a third angle.

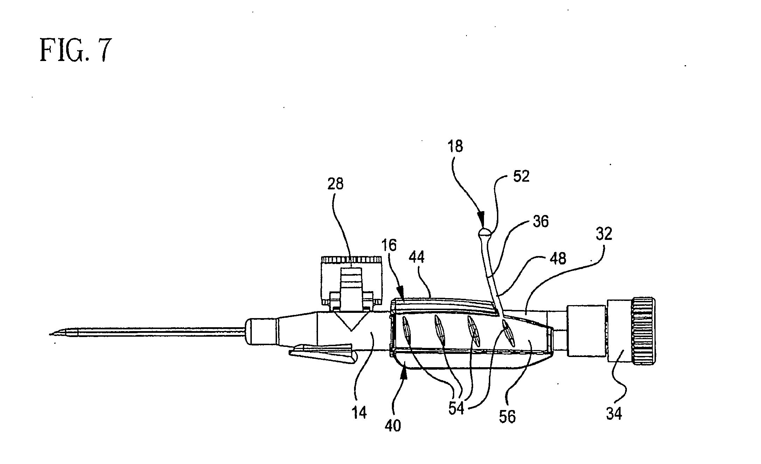

FIG. 7 is an alternative embodiment with a support rib removed from the needle grip.

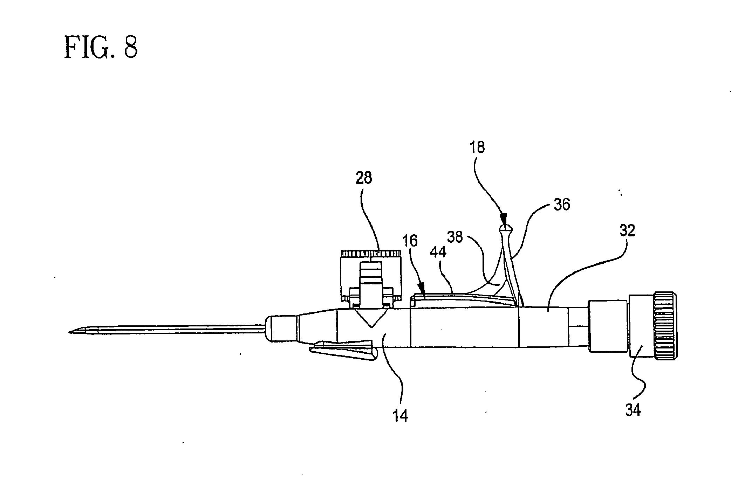

FIG. 8 is another alternative embodiment with a first side grip and a second side grip removed from the needle grip.

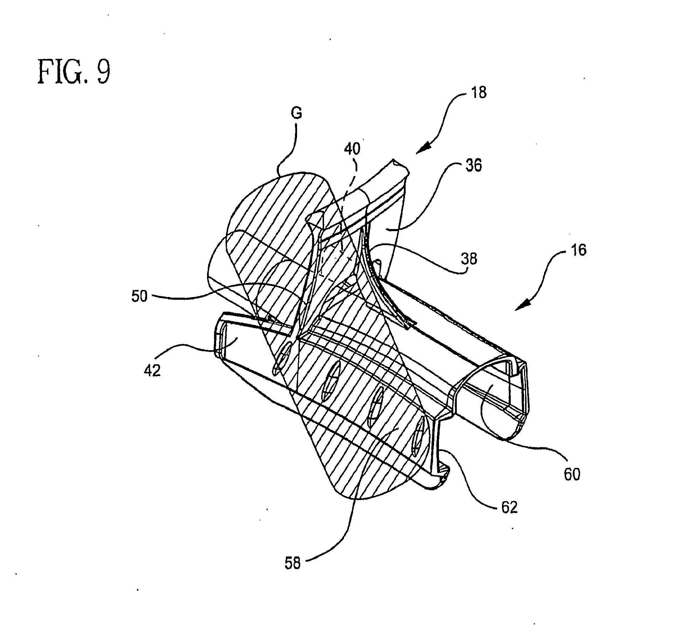

FIG. 9 is a perspective view of the needle hub detached from the catheter assembly.

### DETAILED DESCRIPTION

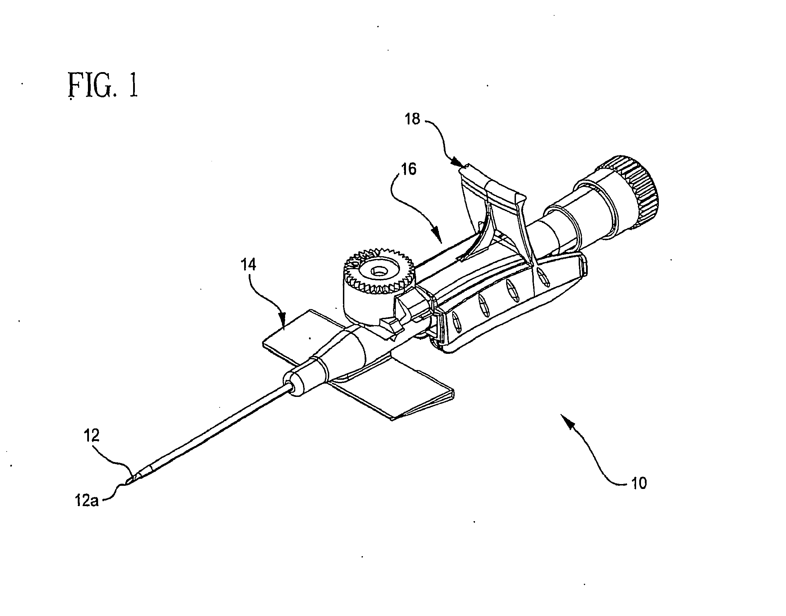

FIG. 1 is a representative embodiment of catheter assembly 10 prior to placement in a vein. Catheter assembly 10 includes needle 12 with needle tip 12a, catheter adapter 14, and needle hub 16 with needle grip 18. The distal end of catheter assembly 10 is generally toward needle tip 12a and the proximal end is generally toward needle hub 16.

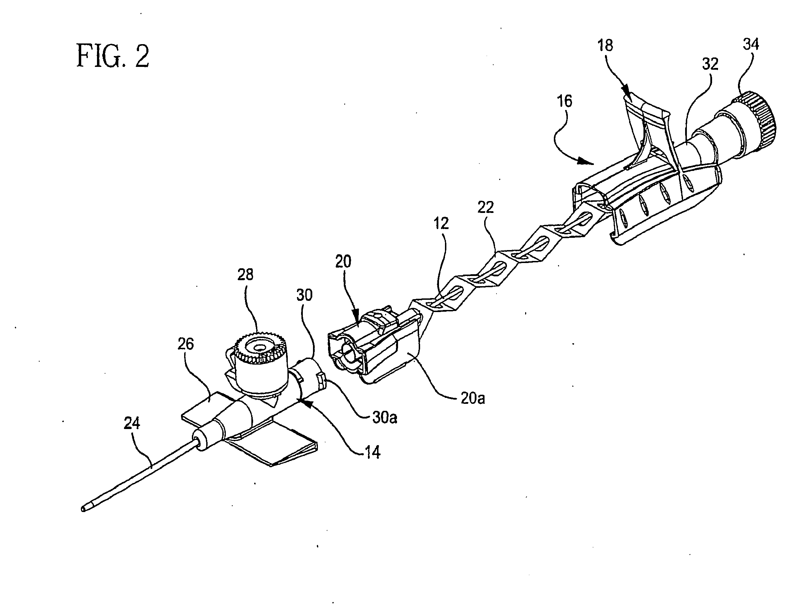

FIG. 2 shows catheter assembly 10 after placement into a vein. In addition to the elements shown in FIG. 1, FIG. 2 includes tip shield 20 with housing 20a and tether 22. Catheter adapter 14 includes catheter 24, wings 26, injection port 28, and notch 30 with luer lock member 30a. Needle hub 16 includes flashback chamber 32 and flow control plug 34.

In use, needle 12 of the assembled catheter assembly 10 shown in FIG. 1 is inserted at an angle through the patient's skin into a vein. Placement of needle 12 with catheter 24 in a vein is verified by confirming that there is a flashback of blood in flashback chamber 32. Once confirmed, pressure is applied to the vein distal of needle 12 by pressing down on the patient's skin. This pressure occludes or at least minimizes further blood flow through needle 12 and catheter 24. Needle 12 is then withdrawn from catheter 24 by pulling needle hub 16 in a proximal direction from catheter adapter 14 leaving catheter 24 in place in the patient's vein.

As needle 12 is withdrawn from catheter 24, needle hub 16 pulls away in a proximal direction from tip shield 20. As this occurs, tether 22 unfolds to extend between tip shield 20 and needle hub 16. When needle tip 12a is withdrawn into tip shield 20, tip shield 20 disengages from catheter adapter 14. At this point, tip shield 20 now covers needle tip 12a to prevent accidental needle sticks. The length of tether 22 is such that when fully extended, tip shield 20 encompasses needle tip 12a. Tether 22 prevents tip shield 20 from falling off needle tip 12a. The clinician is then able to discard needle 12. It should be noted that tip shield 20 shown and described in reference to FIG. 2 is only one possible embodiment of a tip shield that may be used in catheter assembly 10. For example, a tip shield may be designed without incorporating a tether.

Prior to insertion of catheter 24 into the patient, tip shield 20 of catheter assembly 10 is encased by needle hub 16. One benefit of such a design is to minimize the risk of early withdrawal of needle 12 from catheter 24. Even when catheter assembly 10 is held improperly by some portion other than needle grip 18, the placement of tip shield 20 within needle hub 16 minimizes the possibility that the clinician's grasp on catheter assembly 10 will cause a premature separation of needle hub 16 from tip shield 20, thereby causing needle 12 to withdraw. In addition, the risk of separating needle hub 16 from tip shield 20 and causing, for example, tether 22 to tear is also minimized.

As shown in FIG. 2, catheter adapter 14 also includes wings 26 and port 28. Wings 26 provide an anchor for catheter 24 upon insertion into the patient. Wings 26 are large enough to allow taping or other means of attachment to secure catheter adapter 14 to the patient so that once catheter 24 is inserted, it does not move or withdraw from the patient's body unintentionally. Port 28 provides an input to catheter adapter 14 and allows connection to such things as medicine or fluids to catheter 24. In addition, wings 26 function to reduce pressure on the patient when injecting the patient with a fluid through injection port 28.

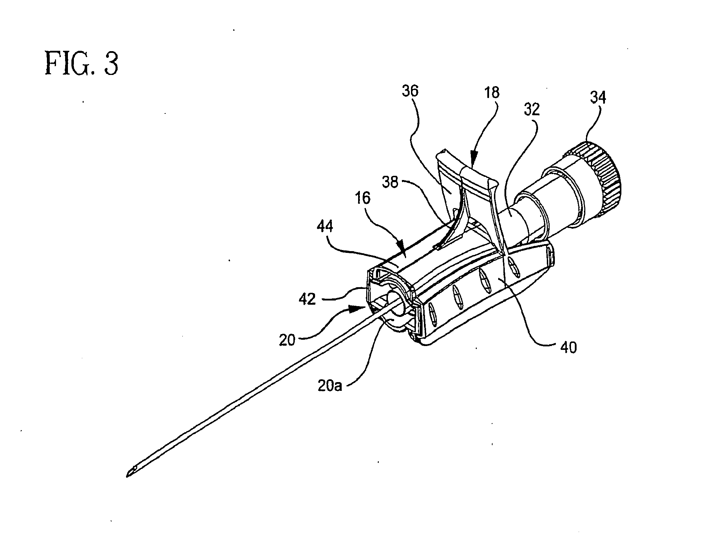

FIG. 3 is a detailed perspective view of needle grip 18. Needle grip 18 includes grip plate 36, support rib 38, first side grip 40, and second side grip 42. As shown in FIG. 3, support rib 38 is attached on one end to grip plate 36, and on a second end to outer surface 44 of needle hub 16. The purpose of support rib 38 is to reinforce grip plate 36 and to provide additional stiffness so that when the clinician's thumb is positioned on grip plate 36 and a force is supplied onto grip plate 36 by the thumb during insertion, grip plate 36 will not bend.

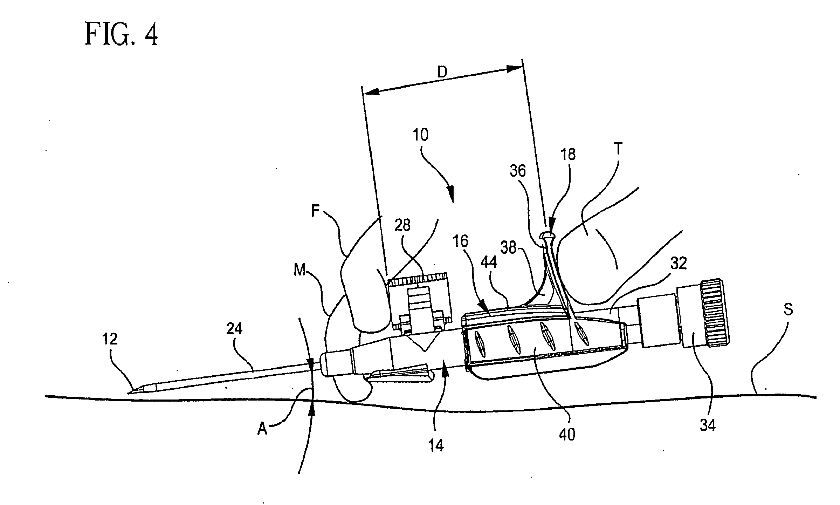

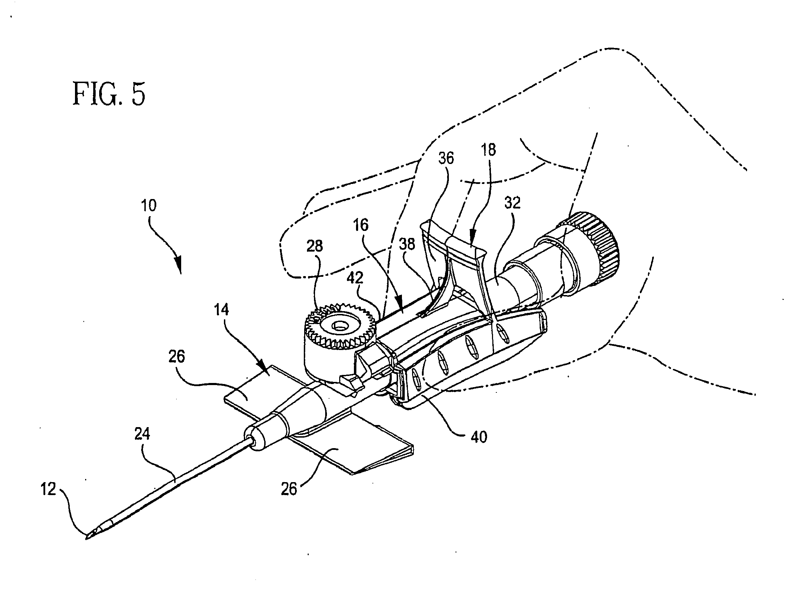

As shown in FIGS. 1 and 2, catheter assembly 10 is a ported catheter. Clinicians who are accustomed to inserting ported catheters will generally grasp catheter assembly 10 by grip plate 36, injection port 28, and a front edge of one of wings 26 while inserting catheter 24 into a patient. This insertion technique is shown in FIG. 4. Alternatively, clinicians may grasp catheter assembly 10 by grip plate 36 and injection port 28. On the other hand, clinicians who are accustomed to inserting straight catheters will generally grasp catheter assembly 10 by first side grip 40 and second side grip 42. This insertion technique is shown in FIG. 5. Thus, needle grip 18 of the present invention is adapted to accommodate either insertion technique.

FIG. 4 is a side view of catheter assembly 10 showing a "ported catheter" technique for grasping needle grip 18. As shown in FIG. 4, angle A is formed between catheter 24 and skin surface S. Angle A is generally small in value so that catheter 24 is almost parallel with skin surface S. In FIG. 4, the clinician grasps catheter assembly 10 by placing thumb T on the proximal side of grip plate 36, forefinger F on the distal side of injection port 28, and middle finger M on the distal side of one of wings 26. Grip plate 36 is angled toward the distal end of catheter assembly 10, thereby providing an angled thumb position. Because grip plate 36 is distally inclined relative to needle 12, thumb T is in a more comfortable, ergonomic position during the insertion of needle 12 and catheter 24 into the patient. Even a slight angled thumb position is beneficial.

The angled thumb position provides a secure grip on grip plate 36 not only when angle A is small, but also when catheter assembly 10 is rotated to various angles A. Some clinicians prefer to begin the insertion process so that angle A has a value close to 90 degrees. But, even with that technique, once the clinician inserts needle 12 and catheter 24 into the patient's vein, the clinician typically rotates catheter assembly 10 down such that angle A gradually decreases until catheter 24 is almost parallel with skin surface S. Thus, whether angle A is large or small to begin with, angle A will typically end up small in value such that catheter 24 is almost parallel with skin surface S. As a result, regardless of the clinician's insertion technique, the angled grip plate 36 of the present invention provides a comfortable, secure, and ergonomic grip for the clinician.

Another feature of catheter assembly 10 that helps provide a comfortable, secure, and ergonomic grip is an optimized distance D between injection port 28 and grip plate 36. In particular, distance D is measured from a distal side of injection port 28 to an upper edge of grip plate 36. Since forefinger F and thumb T are typically placed on injection port 28 and grip plate 36, respectively, distance D represents an optimized distance between the clinician's forefinger F and thumb T that reduces muscular stress during insertion of needle 12 and catheter 24. Thus, in addition to angled grip plate 36, distance D between injection port 28 and grip plate 36 also provides a comfortable, secure, and ergonomic grip for the clinician. In a preferred embodiment, distance D is about 27 millimeters. However, distance D may have a value within a range of about 17 millimeters to 37 millimeters without departing from the spirit and scope of the present invention.

FIG. 5 is a perspective view of catheter assembly 10 showing a "straight catheter" technique for grasping needle grip 18. To insert needle 12 and catheter 24 into the patient using first side grip 40 and second side grip 42, the clinician typically places a thumb on first side grip 40 and a forefinger of the same hand on second side grip 42, or vice versa. As shown in FIG. 5, the clinician is utilizing an increased gripping surface provided by needle grip 18 by placing the forefinger against both grip plate 36 and second side grip 42. As a result of their position, shape, and contours, grip plate 36, first side grip 40, and second side grip 42 provide a comfortable, ergonomic, and secure gripping surface for the clinician.

FIG. 6*a* is a side view of needle grip 18 with grip plate 36 at first preferred angle X. In FIG. 6*a*, angle X is formed between grip plate 36 and axis 46 extending longitudinally along a centerline of needle 12. Angle X is measured from a point on axis 46 near the proximal end of needle hub 16. In the preferred embodiment shown in FIG. 6*a*, angle X is 105 degrees. When grasping needle hub 16 for insertion as described above with reference to FIG. 4, the clinician's thumb is placed on grip plate 36. During the preferred method of insertion, needle 12 is almost parallel to skin surface S (FIG. 4). Thus, during the insertion process, the clinician's thumb remains at an angle approximately equal to angle X. This angled thumb position provides comfort to the clinician and ensures a secure grip on grip plate 36 even when needle 12 is rotated such that it is almost parallel to skin surface S.

FIG. 6*b* is a side view of needle grip 18 with grip plate 36 at second preferred angle Y. In FIG. 6*b*, angle Y is formed between grip plate 36 and axis 46 extending longitudinally along the centerline of needle 12. Angle Y is measured from a point on axis 46 near the proximal end of needle hub 16. In this embodiment, angle Y is slightly over 90 degrees. When grasping needle hub 16 for insertion as described above with reference to FIG. 4, the clinician's thumb is placed on grip plate 36. During the preferred method of insertion, needle 12 is almost parallel to skin surface S (FIG. 4). Thus, during the insertion process, the clinician's thumb remains at an angle approximately equal to angle Y. Second angle Y is smaller than angle X, but positioning grip plate 36 at angle Y still provides comfort and a secure grip.

FIG. 6*c* is a side view of needle grip 18 with grip plate 36 at third preferred angle Z. In FIG. 6*c*, angle Z is formed between grip plate 36 and axis 46 extending longitudinally along the centerline of needle 12. Angle Z is measured from a point on axis 46 near the proximal end of needle hub 16. In this embodiment, angle Z is 120 degrees. Once again, when grasping needle hub 16 for insertion as described above with reference to FIG. 4, the clinician's thumb is placed on grip plate 36. During the preferred method of insertion, needle 12 is almost parallel to skin surface S (FIG. 4). Thus, during the insertion process, the clinician's thumb remains at an angle approximately equal to angle Z. Once again, this angled thumb position provides comfort to the clinician and ensures a secure grip on grip plate 36 even when needle 12 is rotated such that it is almost parallel to skin surface S. As indicated by the range of angles in FIGS. 6*a**,* 6*b**,* and 6*c*, the preferred range of angles of grip plate 36 is greater than 90 degrees but less than or equal to 120 degrees, although any grip plate position that forms an obtuse angle with a point on axis 46 near the proximal end of needle hub 16 still provides comfort and a secure grip and is within the intended scope of the invention.

FIG. 7 is a side view of an alternative embodiment with support rib 38 removed from needle grip 18. It should be understood that while support rib 38 is not required, it does provide additional reinforcement and is generally advantageous when a grip plate material lacks sufficient stiffness.

Furthermore, in the embodiment shown in FIG. 7, first side grip 40 and second side grip 42 extend up first side 48 and second side 50 (not shown) of grip plate 36. In FIG. 7, second side grip 42 is positioned directly opposite first side grip 40 and is therefore not visible. However, because first side grip 40 and second side grip 42 are mirror images of each other in the embodiment shown in FIG. 7, the foregoing discussion with respect to first side grip 40 applies in the same manner to second side grip 42.

First side grip 40 couples with first side 48 of grip plate 36, together creating a firm and ergonomic grip surface with a large surface area for insertion or removal of needle 12 and catheter 24. Because flashback chamber 32 is located near the proximal end of needle hub 16, a digit of the clinician placed upon grip plate 36, first side grip 40, or second side grip 42 during insertion of needle 12 and catheter 24 into the patient does not interfere with the visual indication of blood in flashback chamber 32 (which indicates proper insertion into the vein).

Grip plate 36 further includes lip 52 located on the outer end of grip plate 36. When the clinician grasps catheter assembly 10 by needle grip 18, lip 52 on grip plate 36 helps to prevent the clinician's thumb from slipping off of grip plate 36 during insertion or removal. Furthermore, first side grip 40 and second side grip 42 both contain a plurality of ridges 54 located on outer surface 56 of first side grip 40 and outer surface 58 of second side grip 42 (not shown). Ridges 54 serve multiple purposes, including but not limited to a visual signal where to grip catheter assembly 10, and a feature to help prevent the clinician's thumb and other fingers from slipping off of first side grip 40 and second side grip 42 during insertion or removal. Although first and second side grips 40 and 42 have been described as including a plurality of ridges 54, other gripping features including but not limited to a plurality of bumps, an over-molded soft gripping material, or a rough side grip surface may replace ridges 54 without departing from the intended scope of the present invention.

FIG. 8 is a side view of another alternative embodiment with first side grip 40 and second side grip 42 removed from needle grip 18. In such an embodiment, the clinician typically grasps catheter assembly 10 by grip plate 36 and injection port 28, or alternatively by opposing sides of needle hub 16.

FIG. 9 is a perspective view of needle hub 16 detached from catheter assembly 10. Note that the view shown in FIG. 9 is reversed from previous figures such that "distal" is to the right and "proximal" is to the left. Second side grip 42 couples with second side 50 of grip plate 36 to create a firm, continuous, and ergonomic grip surface for insertion or removal of needle 12. Specifically, second side 50 of grip plate 36 flows into and attaches directly to second side grip 42, thereby increasing the total gripping surface for the clinician. First side grip 40 couples with first side 48 of grip plate 36 in the exact same manner to increase the total gripping surface on the side of needle hub 16 to which first side grip 40 is attached. The shaded area in FIG. 9 represents an example of an increased gripping surface G created by second side grip 42 and second side 50 of grip plate 36. This increased gripping surface results from the fact that second side grip 42 flows into and extends up second side 50 of grip plate 36, thereby creating one continuous surface for the clinician's finger or thumb.

Needle hub 16 further comprises inner hub surface 60 and inner hub surface 62. Inner hub surface 62 is not visible in FIG. 9, but it is located behind outer surface 58 of second side grip 42 and mirrors inner hub surface 60. Inner hub surfaces 60 and 62 extend longitudinally along an interior of needle hub 16. As shown in FIG. 3, inner hub surfaces 60 and 62 couple with housing 20a of tip shield 20 to form an anti-rotation feature. The anti-rotation feature is configured to ensure grip stability of catheter assembly 10 during use by the clinician. In particular, inner hub surfaces 60 and 62 are configured to receive a pair of protrusions extending along housing 20a such that tip shield 20 is unable to rotate when encased by needle hub 16. Since needle hub 16 is unable to rotate relative to tip shield 20 during insertion or removal of catheter 24 and needle 12, catheter assembly 10 provides a stable, secure grip for the clinician.

In the preferred embodiment, grip plate 36, first side grip 40, and second side grip 42 are all formed from a clear material. Furthermore, a preferred clear material for creating these components of needle grip 18 is polypropylene. However, various other plastics, both clear and opaque, may be used without departing from the scope of the present invention.

The present invention provides a catheter assembly with an ergonomic needle grip that enables the clinician to grasp the catheter assembly securely, and that further allows for varying insertion and removal techniques. The design of the present invention includes a grip plate attached to and extending radially from an outer surface of a needle hub of the catheter assembly. The grip plate forms an obtuse angle with an axis extending longitudinally along a centerline of a needle attached to the needle hub, thereby providing a comfortable, secure, and ergonomic gripping surface, as well as increased visibility of a flashback chamber in the needle hub.

Although the present invention has been described with reference to preferred embodiments, workers skilled in the art will recognize that changes may be made in form and detail without departing from the scope of the invention.

## Claims

1. A catheter assembly comprising:
a needle hub (16) having an outer surface, a distal end, and a proximal end;
a grip plate (36) attached to and extending radially from the outer surface of the needle hub (16),
**characterized in that**
the grip plate (36) forms an obtuse angle with an axis (46) extending longitudinally along a centerline of a needle of the catheter assembly, measured from a point on the axis (46) near the proximal end of the needle hub (16).
wherein the grip plate(36) is configured and positioned to receive a user's thumb such that the user's thumb applies a pressure in a direction of the distal end of the needle hub (16) for advancing a catheter.

2. The catheter assembly of claim 1, and further comprising a first side grip (40) attached to the needle hub (16) longitudinally along the outer surface of the needle hub (16).

3. The catheter assembly of claim 2, and further comprising a second side grip (42) attached to the needle hub (16) longitudinally along the outer surface of the needle hub (16).

4. The catheter assembly of claim 3, wherein the first side grip (40) extends up a first side (48) of the grip plate (36), and the second side grip (42) extends up a second side (50) of the grip plate (36).

5. The catheter assembly of claim 4, wherein the first side grip (40) and the second side grip (42) comprise clear material.

6. The catheter assembly of claim 4, wherein the first side grip (40), the second side grip (42), and the grip plate (36) comprise polypropylene.

7. The catheter assembly of claim 4, and further comprising a gripping feature on an outer surface of the first side grip (40) and an outer surface of the second side grip (42) configured to provide a secure gripping surface.

8. The catheter assembly of claim 1, wherein the angle formed by the grip plate (36) and the axis (46) is greater than 90 degrees and less than or equal to about 120 degrees.

9. The catheter assembly of claim 8, wherein the angle formed by the grip plate (36) and the axis (46) is about 105 degrees.

10. The catheter assembly of claim 1, and further comprising a support rib (38) attached to the grip plate (36).

11. The catheter assembly of claim 1, wherein the grip plate (36) includes a lip (52).

12. The catheter assembly of claim 1, where a flashback chamber (32) of the catheter assembly located near the proximal end of the needle hub (16) is visible when a thumb is placed on the grip plate (36).

13. The catheter assembly of claim 2 or 3, further comprising a plurality of ridges (54) on the outer surface of the first side grip (40) and the outer surface of the second side grip (42).

14. The catheter assembly of claim 1, further comprising a catheter adapter (14) having an injection port (28), wherein a distance between a distal side of the injection port (28) and an upper edge of the grip plate (36) is between about 17 millimeters and about 37 millimeters.

15. The catheter assembly of claim 14, wherein the distance is about 27 millimeters.

16. The catheter assembly of claim 1, further comprising a tip shield connectable to the needle hub (16).

17. The catheter assembly of claim 16, wherein the needle hub (16) further comprises:
a first inner hub surface (60); and
a second inner hub surface (62), wherein the first and second inner hub surfaces (60, 62) are each configured to receive a protrusion extending along a housing (20a) of the tip shield to prevent the tip shield from rotating with respect to the needle hub (16) when the tip shield is connected to the needle hub (16).

## Patentansprüche

1. Katheteranordnung mit:
einem Nadelansatz (16) mit einer Außenfläche, einem distalen Ende und einem proximalen Ende;
einer Griffplatte (36), die an der Außenfläche des Nadelansatzes (16) angebracht ist und sich radial von dieser erstreckt,
**dadurch gekennzeichnet, dass**
die Griffplatte (36) einen stumpfen Winkel mit einer Achse (46) bildet, die sich in Längsrichtung entlang einer Mittellinie einer Nadel der Katheteranordnung erstreckt, wobei der Winkel von einem nahe dem proximalen Ende des Nadelansatzes (16) gelegenen Punkt auf der Achse (46) gemessen ist,
wobei die Griffplatte (36) derart ausgebildet und positioniert ist, dass sie den Daumen eines Benutzers derart aufnimmt, dass der Daumen des Benutzers einen Druck in Richtung des distalen Endes des Nadelansatzes (16) aufbringt, um einen Katheter vorzubewegen.

2. Katheteranordnung nach Anspruch 1, und ferner mit einem ersten seitlichen Griff (40), welcher an dem Nadelansatz (16) in Längsrichtung entlang der Außenfläche des Nadelansatzes (16) angebracht ist.

3. Katheteranordnung nach Anspruch 2, und ferner mit einem zweiten seitlichen Griff (42), welcher an dem Nadelansatz (16) in Längsrichtung entlang der Außenfläche des Nadelansatzes (16) angebracht ist.

4. Katheteranordnung nach Anspruch 3, bei welcher der erste seitliche Griff (40) auf einer ersten Seite (48) der Griffplatte (36) erstreckt, und der zweite seitliche Griff (42) sich auf einer zweiten Seite (50) der Griffplatte (36) erstreckt.

5. Katheteranordnung nach Anspruch 4, bei welcher der erste seitliche Griff (40) und der zweite seitliche Griff (42) durchsichtiges Material aufweisen.

6. Katheteranordnung nach Anspruch 4, bei welcher der erste seitliche Griff (40), der zweite seitliche Griff (42) und die Griffplatte (36) Polypropylen aufweisen.

7. Katheteranordnung nach Anspruch 4, und ferner mit einer Greifeinrichtung auf einer Außenfläche des ersten seitlichen Griffs (40) und einer Außenfläche des zweiten seitlichen Griffs (42) zur Bildung einer sicheren Greiffläche.

8. Katheteranordnung nach Anspruch 1, bei welcher der von der Griffplatte (36) und der Achse (46) gebildete Winkel größer als 90 Grad und kleiner als oder gleich ungefähr 120 Grad ist.

9. Katheteranordnung nach Anspruch 8, bei welcher der von der Griffplatte (36) und der Achse (46) gebildete Winkel ungefähr 105 Grad beträgt.

10. Katheteranordnung nach Anspruch 1, und ferner mit einer an der Griffplatte (36) angebrachten Stützrippe (38).

11. Katheteranordnung nach Anspruch 1, bei welcher die Griffplatte (36) eine Lippe (52) aufweist.

12. Katheteranordnung nach Anspruch 1, bei welcher eine nahe dem proximalen Ende des Nadelansatzes (16) angeordnete Rückflusskammer (32) der Katheteranordnung sichtbar ist, wenn ein Daumen auf der Griffplatte (36) platziert ist. ,

13. Katheteranordnung nach Anspruch 2 oder 3, ferner mit mehreren Graten (54) auf der Außenfläche des ersten seitlichen Griffs (40) und der Außenfläche des zweiten seitlichen Griffs (42).

14. Katheteranordnung nach Anspruch 1, ferner mit einem Katheteradapter (14), der eine Injektionsöffnung (28) aufweist, wobei der Abstand zwischen der distalen Seite der Injektionsöffnung (28) und dem oberen Rand der Griffplatte (36) zwischen ungefähr 17 Millimeter und ungefähr 37 Millimeter beträgt.

15. Katheteranordnung nach Anspruch 14, bei welcher der Abstand ungefähr 27 Millimeter beträgt.

16. Katheteranordnung nach Anspruch 1, ferner mit einem mit dem Nadelansatz (16) verbindbaren Spitzenschutz.

17. Katheteranordnung nach Anspruch 16, bei welcher der Nadelansatz (16) ferner aufweist:
eine erste Ansatzinnenfläche (60); und
eine zweite Ansatzinnenfläche (62), wobei die erste und die zweite Ansatzinnenfläche (60, 62) jeweils zur Aufnahme eines entlang eines Gehäuses (20a) des Spitzenschutzes verlaufenden Vorsprungs ausgebildet sind, um das Drehen des Spitzenschutzes in Bezug auf den Nadelansatz (16) zu verhindern, wenn der Spitzenschutz mit dem Nadelansatz (16) verbunden ist.

## Revendications

1. Ensemble de catheter comprenant:
un embout d'aiguille (16) comprenant une face externe, une extrémité distale et une extrémité proximale;
une plaque de prise (36) attachée à et s'étendant radialement à partir de ladite face externe dudit embout d'aiguille (16),
**caractérisé en ce que**
ladite plaque de prise (36) forme un angle obtus avec un axe (46) s'étendant suivant une ligne centrale d'une aiguille dudit ensemble de cathéter, mesuré à partir d'un point sur l'axe (46) près de ladite extrémité proximale dudit embout d'aiguille (16),
ladite plaque de prise (36) étant configurée et positionnée pour recevoir le pouce d'un utilisateur de sorte que ledit pouce de l'utilisateur applique une pression dans la direction de ladite extrémité distale dudit embout d'aiguille (16) pour faire avancer un cathéter.

2. Ensemble de cathéter selon la revendication 1, et comprenant en outre un premier moyen de prise latéral (40) attaché audit embout d'aiguille (16) suivant la direction longitudinale de ladite face externe dudit embout d'aiguille (16).

3. Ensemble de cathéter selon la revendication 2, et comprenant en outre un deuxième moyen de prise latéral (42) attaché audit embout d'aiguille (16) suivant la direction longitudinale de ladite face externe dudit embout d'aiguille (16).

4. Ensemble de cathéter selon la revendication 3, dans lequel ledit premier moyen de prise latéral (40) s'étend sur un premier côté (48) de ladite plaque de prise (36), et ledit deuxième moyen de prise latéral (42) s'étend sur un deuxième côté (50) de ladite plaque de prise (36).

5. Ensemble de cathéter selon la revendication 4, dans lequel ledit premier moyen de prise latéral (40) et ledit deuxième moyen de prise latéral (42) comprennent une matière claire.

6. Ensemble de cathéter selon la revendication 4, dans lequel ledit premier moyen de prise latéral (40), ledit deuxième moyen de prise latéral (42) et ladite plaque de prise (36) comprennent du polypropylène.

7. Ensemble de cathéter selon la revendication 4, et comprenant en outre un élément de préhension sur une face externe dudit premier moyen de prise latéral (40) et une face externe dudit deuxième moyen de prise latéral (42), configuré pour former une face de préhension fiable.

8. Ensemble de cathéter selon la revendication 1, dans lequel ledit angle formé par ladite plaque de prise (36) et ledit axe (46) est supérieur à 90 dégrées et inférieur ou égal à environ 120 dégrées.

9. Ensemble de cathéter selon la revendication 8, dans lequel ledit angle formé par ladite plaque de prise (36) et ledit axe (46) est environ 105 dégrées.

10. Ensemble de cathéter selon la revendication 1, et comprenant en outre une nervure (38) prévue sur ladite plaque de prise (36).

11. Ensemble de cathéter selon la revendication 1, dans lequel ladite plaque de préhension (36) comprend une pointe (52).

12. Ensemble de cathéter selon la revendication 1, dans lequel une chambre de reflux (32) dudit ensemble de cathéter, située près de ladite extrémité proximale dudit embout d'aiguille (16), est visible quand un pouce est placé sur ladite plaque de préhension (36).

13. Ensemble de cathéter selon les revendications 2 ou 3, comprenant en outre plusieurs arêtes (54) sur la face externe dudit premier moyen de prise latéral (40) et la face externe dudit deuxième moyen de prise latéral (42).

14. Ensemble de cathéter selon la revendication 1, comprenant en outre un adaptateur de cathéter (14) avec une ouverture d'injection (28), la distance entre le côté distal de ladite ouverture d'injection (28) et un bord supérieur de ladite plaque de prise (36) se situe entre environ 17 millimètres et environ 37 millimètres.

15. Ensemble de cathéter selon la revendication 14, dans lequel la distance est environ 27 millimètres.

16. Ensemble de cathéter selon la revendication 1, comprenant en outre un protecteur de pointe apte à être connecté audit embout d'aiguille (16).

17. Ensemble de cathéter selon la revendication 16, dans lequel ledit embout d'aiguille (16) comprend en outre:
une première face interne d'embout (60); et
une deuxième face interne d'embout (62), ladite première et ladite deuxième face interne d'embout (60, 62) étant chacune configurée pour recevoir une saillie s'étendant le long d'un boitier (20a) dudit protecteur de pointe pour empêcher la rotation dudit protecteur de pointe par rapport audit embout d'aiguille (16) quand ledit protecteur de pointe est connecté audit embout d'aiguille (16).
